# EUROPÄISCHE PATENTSCHRIFT

(11) **EP 3 285 930 B1**
(45) Veröffentlichungstag und Bekanntmachung des Hinweises auf die Patenterteilung: **25.03.2020**
(21) Anmeldenummer: 16723944.1
(22) Anmeldetag: 22.04.2016
(51) Int. Cl.: B01L 9/00, B01L 9/06, C12M 3/00, G01N 35/00, C12M 1/00, C12M 1/22

(54) **TRÄGER ZUM AUFNEHMEN UND LAGERN VON LABORGEFÄSSEN**
HOLDER FOR RECEIVING AND STORING LABORATORY VESSELS
SUPPORT POUR LA RÉCEPTION ET POUR LE RANGEMENT DE RÉCIPIENTS DE LABORATOIRE

(30) Priorität: 24.04.2015 DE 102015207617
(43) Veröffentlichungstag der Anmeldung: 28.02.2018
(73) Patentinhaber: Andreas Hettich GmbH & Co. KG, 78532 Tuttlingen (DE)
(72) Erfinder: EBERLE, Klaus-Günter, 78532 Tuttlingen (DE)
(74) Vertreter: Puschmann Borchert Bardehle Patentanwälte Partnerschaft mbB
(86) Internationale Anmeldenummer: PCT/EP2016/059089
(87) Internationale Veröffentlichungsnummer: WO 2016/170161

(56) Entgegenhaltungen:
- EP-A1- 1 018 544
- EP-A1- 1 445 309
- DE-U1-202005 017 383
- US-A- 3 830 701
- US-A- 4 143 765
- US-A1- 2006 211 080
- US-A1- 2012 175 328

## Beschreibung

Die Erfindung betrifft einen Träger zum Aufnehmen und Lagern von Laborgefäßen gemäß der im Oberbegriff des Patentanspruches 1 angegebenen Art.

Im Stand der Technik existiert eine Vielzahl von Tragvorrichtungen, in denen Laborgefäße, insbesondere Petrischalen, transportiert und gelagert werden.

Petrischalen sind auf Grund ihrer Form gut zum Stapeln geeignet. In Inkubatoren, aber auch in komplexeren Vorrichtungen zur Analyse und Auswertung von Proben, können gewöhnlich mehrere Schalenstapel zugleich in dafür vorgesehenen Magazinen gelagert sein.

Es sind verschiedene Petrischalen-Träger bekannt, bei denen ein Gehäuse vier sternförmig zueinander angeordnete zueinander parallele Längsachsen aufweisende Aufnahmen umfasst. Zur Lagerung und zum Transport werden Petrischalen von oben in die Aufnahmen geladen und darin konzentrisch übereinander gestapelt. Nach dem Transport erfolgt die Entnahme der Petrischalen ebenso von oben. Die Aufnahmen sind teilkreisförmig ausgebildet und an den voneinander weg weisenden Seiten geöffnet. Dabei erstrecken sich die Öffnungen in der Regel über weniger als die Hälfte des Kreisbogens, so dass die eingebrachten Petrischalen nicht herausfallen können.

In der Praxis erweist sich die Be- und Entladung solcher Träger jedoch immer wieder als unpraktisch. Die Petrischalen müssen einzeln oder in kleineren Gruppen aus einer Aufnahme nach der anderen entnommen bzw. in diese eingebracht werden. Mit steigender Höhe eines Stapels von Petrischalen, die gleichzeitig bewegt werden sollen, nimmt dabei auch das Risiko zu, dass der Stapel instabil wird und Petrischalen verrutschen oder gar herunterfallen. Oft erfolgt diese Tätigkeit zudem manuell, was für den Benutzer monoton und anstrengend zugleich ist.

Aus der US 2006/0211080 A1 ist ein gattungsgemäßer Träger bekannt, bei dem ein Schieber eine Entladeöffnung verschließt und öffnet und gleichzeitig eine Petrischale seitlich translatorisch verschiebt. Eine derartige Konstruktion baut sehr groß und ist im Einsatzbereich eingeschränkt.

Die EP 1 445 309 A1 offenbart eine seitliche Entladeöffnung eines Magazins für Petrischalen,

Die US 3 830 701 B offenbart eine Vorrichtung zum Transport und Sammeln von Petrischalen. Dabei ist die Ladeöffnung im unteren Bereich angeordnet.

Die EP 1 018 544 A1 offenbart zum Schließen der Ladeöffnung kippbare Klinken.

Aus der EP 0 242 114 A2 ist ein Träger zum Aufnehmen und Lagern von Laborgefäßen bekannt. Die Entladeöffnung ist mit Sperrklinken versehen, welche ein Herausbewegen der Laborgefäße aus dem Träger verhindern. Die Sperrklinken sind durch Federn in die Schließstellung vorgespannt. Die Laborgefäße können durch die Entladeöffnung ausgebracht werden, in dem die Sperrklinke durch einen Mechanismus in die Öffnungsstellung verschwenkt wird und die Entladeöffnung freigibt. Hierfür ist die Sperrklinke als Kipphebel ausgebildet, an die ein Drücker zum Öffnen an dem der Sperrklinke entfernt angeordneten Ende angreift. Hat der Drücker die Sperrklinke freigegeben, wird die Sperrklinke durch die Feder in der Schließstellung gehalten. Von unten kann dann trotzdem gegen die Federkraft der Sperrklinke ein Laborgefäß nach oben unter Überwindung der Federkraft und einer vorbestimmten Hubhöhe, welche im Wesentlichen einem Hebelarm der Sperrklinke entspricht, eingebracht werden, Dieser Sperrklinkenverschließmechanismus ist sehr fehleranfällig und baut sehr groß.

In der EP 0 242 114 A2 wird ein Träger zum Aufnehmen und Lagern von Laborgefäßen offenbart, welche an der unteren Entladeöffnung Sperrklinken aufweist. Die Sperrklinken sind um eine senkrecht zur Entladerichtung verlaufende Drehachse schwenkbar. Die Sperrklinken werden dabei entweder über einen Stift nach oben gedrückt oder mittels einer das Laborgefäß anzuhebenden Hebevorrichtung geöffnet. Dieser Verschließmechanismus ist ebenfalls sehr fehleranfällig und baut sehr groß.

In der DE103 01 446 A1 wird ein Träger zum Aufnehmen und Lagern von Laborgefäßen offenbart. Dieser weist eine einzige Aufnahme für Laborgefäße auf und einen Verschließmechanismus. Dieser Verschließmechanismus, besteht aus schwenkbaren Sperrklinken, welche die Beladeöffnung verschließen, Die Entladeöffnung ist dabei seitlich angeordnet.

Aufgabe der Erfindung ist es, unter Vermeidung der genannten Nachteile einen Träger zum Aufnehmen und Lagern von Laborgefäßen zu schaffen, der ein gleichzeitiges Laden eines oder mehrerer Stapel von in ihm gelagerten Petrischalen in eine geeignete Vorrichtung einerseits und die gleichzeitige Entnahme eines oder mehrerer Stapel von Petrischalen aus einer geeigneten Vorrichtung andererseits bei platzsparender Bauweise ermöglicht.

Diese Aufgabe wird durch die kennzeichnenden Merkmale des Patentanspruches 1 in Verbindung mit seinen Oberbegriffsmerkmalen gelöst.

Die Unteransprüche bilden vorteilhafte Weiterbildungen der Erfindung.

In bekannter Weise umfasst ein Träger zum Aufnehmen und Lagern von Laborgefäßen für Proben, Mikroorganismen, Zellkulturen oder ähnlichem ein Gehäuse und zumindest eine Aufnahme für die Laborgefäße, die sich entlang einer Ladeachse im Gehäuse erstreckt. Die Laborgefäße sind in die Aufnahme einbringbar und darin übereinander stapelbar, und die Aufnahme weist im Gehäuse oben zum Beladen eine Ladeöffnung auf. Die Aufnahme weist auf einer der Ladeöffnung entfernt angeordneten Unterseite eine mittels eines im Gehäuse angeordneten Verschließmechanismus verschließbare Entladeöffnung auf. Dadurch können ein oder mehrere Stapel von im Träger gelagerten Laborgefäßen zugleich in ein Magazin einer Vorrichtung eingebracht und dort nach dem Öffnen des Verschließmechanismus und dem Entfernen des Trägers belassen werden. Ebenso kann ein unbeladener Träger in ein mit einem oder mehreren Stapeln von Laborgefäßen beladenes Magazin einer Vorrichtung eingebracht und nach Verschließen der Verschließvorrichtung mit den Petrischalen entnommen werden. So kann eine große Menge Petrischalen sicher transportiert und auf einmal in ein Magazin oder dergleichen geladen oder aus diesem entnommen werden.Der Verschließmechanismus ist mit einem Schieber versehen, über das der Schieber zum Schließen in die Entladeöffnung in eine Schließposition und zum Öffnen aus der Entladeöffnung in eine Öffnungsposition bewegbar ist. Ein Schieber ist grundsätzlich eine Sperrarmatur, welche in einer Ebene bewegt wird, welche im Wesentlichen senkrecht zum zu sperrenden Materialdurchfluss verläuft, im vorliegenden Fall die Petrischalen, Ein Vorteil eines Schiebers gegenüber den bekannten Sperrklinken ist die flach bauende Ausführung und die Möglichkeit, diesen einfach steuern und antreiben zu können. Verschließmechanismen dieser Art sind wenig fehleranfällig, sind flach bauend und können einfach verbaut werden. Erfindungsgemäß ist der Verschließmechanismus so gestaltet, dass der Schieber aus der Schließposition in die Öffnungsposition und vice versa rotatorisch bewegt wird. So weist die für das Wechseln des Schiebers zwischen der Schließposition und der Öffnungsposition erforderliche Bewegung im Vergleich zur Überdeckung des Laborgefäßes nur einen geringen Winkel auf. Der Verschließmechanismus ist daher sehr effizient.

Gemäß einer Ausführungsform der Erfindung ist ein Getriebe vorgesehen, das mit dem Schieber zusammenwirkt. Hierüber kann die Bewegung des Schiebers exakt eingestellt und angesteuert werden. Zudem ist es möglich, dies mit Automaten oder anderen Einrichtungen zum Be- und Entladen des Trägers zu koppeln.

Vorzugsweise sind mehrere Aufnahmen mit zugeordneten Lade- und Entladeöffnungen vorgesehen, und jeder Entladeöffnung ist ein Schieber zugeordnet. Dadurch kann auf einfache Weise der Träger eine Vielzahl von Stapeln von Laborgefäßen gleichzeitig aufnehmen oder ausgeben. Die Effektivität des Trägers wird hierdurch erhöht.

Die einzelnen Schieber eines Trägers können dabei so miteinander gekoppelt sein, dass über einen Antrieb alle Schieber gleichzeitig bewegbar sind. Hierbei können ohne weiteres mehrere Laborgefäße aus unterschiedlichen Aufnahmen gleichzeitig mit einer Öffnungsbewegung aller Schieber ausgegeben werden. Hierfür ist auch ein gemeinsamer Antrieb vorgesehen. Alternativ kann aber auch eine Ausbildung durch individuelle Ansteuerung der Schieber vorgesehen sein.

Es ist vorteilhaft, wenn der Verschließmechanismus von der Unterseite betätigbar ist, auf der die Entladeöffnung angeordnet ist. Dadurch ist die Betätigung des Verschließmechanismus zum Öffnen und Schließen intuitiv mit der Bewegungsrichtung und dem Zustand - beladen oder unbeladen - des Trägers verbindbar und einfach mit anderen Arbeitsschritten innerhalb einer Vorrichtung koppelbar.

Um eine gute Integration in ein automatisiertes oder teilautomatisiertes System zu erreichen, kann der Verschließmechanismus eine Antriebsnabe oder einen Mitnehmer aufweisen, über die der Antriebsmechanismus motorisch oder manuell, insbesondere mittels einer an die Antriebsnabe oder den Mitnehmer angreifenden Handhabe, betätigbar ist.

Vorzugsweise weist die Aufnahme eine kreisförmige oder zumindest teilkreisförmige Grundfläche auf. So ist sie gut für die Einbringung von Petrischalen geeignet, die häufig in der Biologie und verwandten Bereichen eingesetzt werden und sich gut zur Stapelung eignen.

Gemäß einem weiteren Aspekt der Erfindung sind Ladeöffnung und Entladeöffnung konzentrisch zueinander angeordnet, so dass die Ladeachse sich senkrecht zu der Ladeöffnung und der Entladeöffnung erstreckt und insbesondere parallel zu einer Längsachse des Trägers verläuft. Dadurch finden Beladung, Lagerung und Entladung entlang einer einzelnen Achse statt, und die Gefahr eines unbeabsichtigten Verschiebens oder Verkantens eines Laborgefäßes wird minimiert.

Es ist zweckmäßig, dass ein an der Oberseite des Gehäuses angeordneter Tragegriff vorgesehen ist, der insbesondere im Gehäuse versenkbar gelagert ist. So kann der Träger zum einen bequem am Tragegriff transportiert werden. Zum anderen kann der Träger mit versenktem Tragegriff in kleineren Räumen untergebracht werden, als dies mit einem fixierten Tragegriff möglich wäre. Dies bedeutet eine größere Flexibilität beim Einsatz des Trägers.

Insbesondere kann der Tragegriff vollständig im Gehäuse versenkbar sein, so dass dieser nicht über eine Umhüllende des Gehäuses hinaussteht. Dadurch ist besonders wenig Platz für die Unterbringung des Trägers erforderlich.

In einer bevorzugten Ausgestaltung ist der Träger aus einem hitzebeständigen Werkstoff gebildet, der eine Autoklavierung des Trägers ermöglicht. Dies erleichtert eine Sterilisierung des Trägers.

Gemäß einer Ausführungsform der Erfindung hat es sich als vorteilhaft erwiesen, wenn entlang der Längsachse der Aufnahme für die Laborgefäße wenigstens zur Unterseite des Trägers hin offene Ablaufnuten vorgesehen sind. So können während der Verwendung eintretende Flüssigkeiten leichter abgeleitet werden, und ein Flüssigkeitsstau im Träger wird vermieden.

In einer alternativen Ausgestaltung der Erfindung sind zumindest im Bereich der Entnahmeöffnung und/oder Ladeöffnung Eingriffsausnehmungen vorgesehen, in die Ständer einfahrbar sind, welche mit Abziehen des Trägers die Laborgefäße aufnehmen können. So wird eine einfache, sichere und stabile Entnahme der Laborgefäße erreicht.

Es ist zweckmäßig, dass die Eingriffsausnehmungen als parallel zur Ladeachse ausgerichtete Durchgangsbohrungen oder als zu der Aufnahme für die Laborgefäße hin offene, seitliche Nuten ausgebildet sind. Durch die parallele Ausrichtung zur Ladeachse wird das Risiko eines unbeabsichtigten Verschiebens oder Verkantens der Laborgefäße bei der Entnahme verringert, und die Positionierung der Ständer wird vereinfacht.

Gemäß einem weiteren Aspekt der Erfindung sind zueinander korrespondierende Vorsprünge und Ausnehmungen an der Oberseite und Unterseite vorgesehen, so dass die Vorsprünge und Ausnehmungen bei mehreren gestapelten Trägern ineinandergreifen und dadurch im Wesentlichen normal zur Längsachse formschlüssig fixiert sind. Durch diesen Formschluss wird ein Verrutschen übereinander gestapelter Träger verhindert, und ihre Verwendung wird sicherer.

In einer weiter verbesserten Ausgestaltung sind die Vorsprünge und zugeordneten Ausnehmungen so ausgebildet, dass eine Stapelung mehrerer Gehäuse von Trägern in konzentrischer Weise zueinander ermöglicht wird. Eine konzentrische Stapelung ermöglicht eine optimale Stabilität und eine Minimierung der erforderlichen Grundfläche.

Vorzugsweise ist das Gehäuse als im Kunststoff-Spritzgussverfahren hergestelltes Teil, insbesondere als Vollgussteil, ausgebildet. Ein derartiges Gehäuse ist kostengünstig, einfach den individuellen Anforderungen entsprechend herstellbar und kann leicht gereinigt werden.

Ebenso kann der Tragegriff als in einem Kunststoff-Spritzgussverfahren hergestelltes Teil, insbesondere als Vollgussteil, ausgebildet sein. Auch hier sind niedrige Herstellungskosten und leichte Reinigung ein Vorteil.

Vorzugsweise sind die Ladeöffnung und die Entladeöffnung konzentrisch zueinander angeordnet, insbesondere auch konzentrisch zu einer Beladerichtung und/oder Entladerichtung,

Günstig ist es, wenn seitlich im Gehäuse eine Beschriftungsaufnahme für eine Beschriftungseinlage vorgesehen ist. Dies dient dem Schutz von Beschriftungseinlagen, die zur einfacheren Identifikation eines Trägers durch den Benutzer eingesetzt werden können.

Gemäß einer Ausführungsform der Erfindung hat es sich als vorteilhaft erwiesen, dass für jede Aufnahme eine der jeweiligen Ladeöffnung zugeordnete Kennzeichnung, insbesondere an der Oberseite des Gehäuses, vorgesehenen ist, die die Aufnahme der Laborgefäße eindeutig kennzeichnet. Insbesondere ist die Kennzeichnung in Form einer Ausnehmung oder einer Vertiefung gebildet. So wird eine optische Unterscheidung von Aufnahmen, die mit jeweils unterschiedlichen Proben beladen sind, erleichtert, und entsprechende Aufnahmen mehrerer übereinander gestapelter Träger können einander leichter zugeordnet werden.

In einer bevorzugten Ausführung der Erfindung können die Schieberpaare über ein Getriebe gekoppelt sein, insbesondere in der Art eines Planetengetriebes, und mit einem Antrieb verbindbar sein. Das Getriebe kann Zahnräder umfassen, die in Eingriff mit den Schieberpaaren stehen. Die Zahnräder sind insbesondere auf einem Grundkörper montiert und werden durch den Antrieb in Drehung versetzt. Durch die Drehung des Antriebs werden die Schieberpaare von einer Öffnungsposition in eine Schließposition überführt, die durch Anschläge begrenzt sein können. Als Betätigungsmittel kann eine im Grundkörper zentrierte Vorrichtung vorgesehen sein, welche aus der Zentrierung und einem Ausleger besteht und vorzugsweise mit einem Antrieb verbunden ist, der sich insbesondere an der Be-und Entladestation befindet. So wird ein hoher Automatisierungsgrad des Verschließmechanismus erreicht.

Weitere Vorteile, Merkmale und Anwendungsmöglichkeiten der vorliegenden Erfindung ergeben sich aus der nachfolgenden Beschreibung in Verbindung mit den in den Zeichnungen dargestellten Ausführungsbeispielen.

In der Beschreibung, in den Ansprüchen und in der Zeichnung werden die in der unten aufgeführten Liste der Bezugszeichen verwendeten Begriffe und zugeordneten Bezugszeichen verwendet. In der Zeichnung bedeutet:
- Fig. 1: eine perspektivische Ansicht eines erfindungsgemäßen Trägers;
- Fig. 2a: eine Ansicht von unten auf den Träger mit eingesetzten Petrischalen und in eine Schließposition gebrachten Schiebern;
- Fig. 2b: eine Ansicht von unten auf den Träger mit in eine Öffnungsposition gebrachten Schiebern;
- Fig. 3a: eine perspektivische Ansicht des beladenen Trägers und eines Ständers, und
- Fig. 3b: eine perspektivische Ansicht des Trägers und des beladenen Ständers.

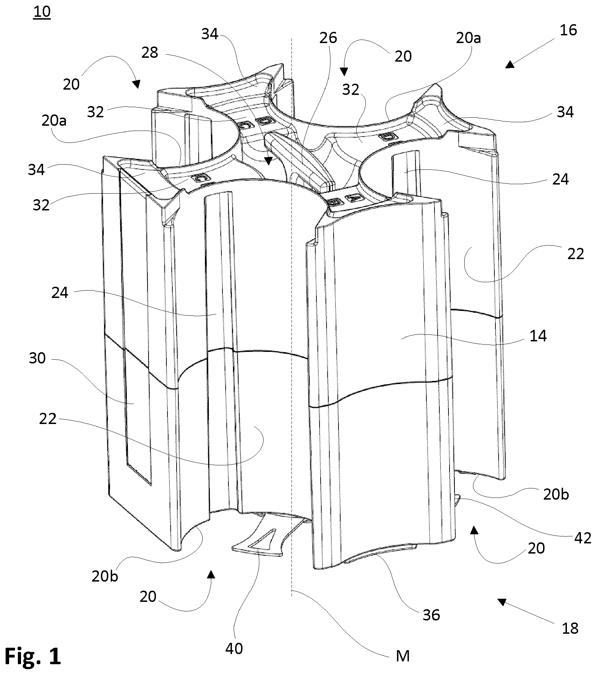

Fig. 1 zeigt eine perspektivische Ansicht eines erfindungsgemäßen Trägers 10 für die Lagerung und den Transport von in Fig. 1 gezeigten Petrischalen.

Der Träger 10 weist ein Gehäuse 14 mit einer Mittelachse M, einer Oberseite 16 und einer der Oberseite 16 gegenüberliegenden Unterseite 18 auf.

In das Gehäuse 14 sind vier sich zwischen der Oberseite 16 und der Unterseite 18 parallel zur Mittelachse M erstreckende Aufnahmen 20 für Petrischalen eingebracht. Jede Aufnahme 20 weist eine durch das Gehäuse 14 ausgebildete Wandung 22 mit einem teilkreisförmigen Querschnitt auf und ist über ihre gesamte Längserstreckung auf der von der Mittelachse M weg weisenden Seite geöffnet. Die Aufnahmen 20 sind sowohl zur Oberseite 16 hin geöffnet, wo sie jeweils eine Ladeöffnung 20a zum Beladen des Trägers 10 mit Petrischalen aufweisen, als auch zur Unterseite 18 hin, wo sie jeweils eine Entladeöffnung 20b zum Entladen der Petrischalen aufweisen. Selbstverständlich können Petrischalen auch über die Ladeöffnung 20a entladen und über die Entladeöffnung 20b beladen werden.

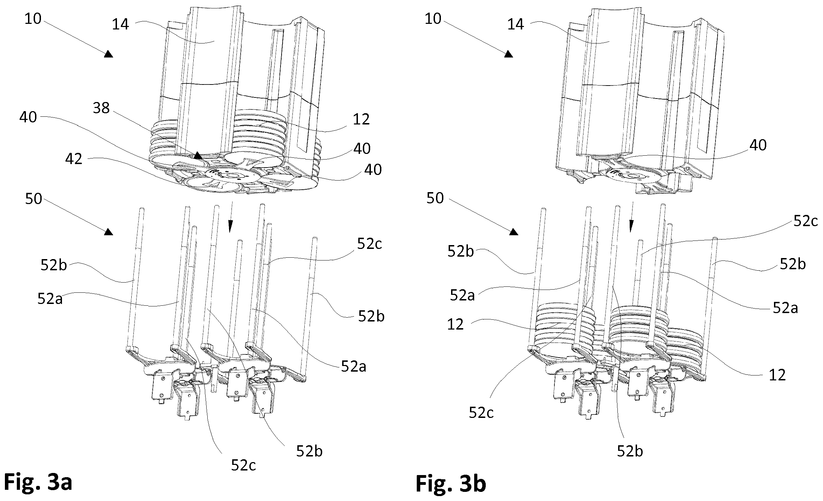

In jeder Wandung 22 sind jeweils zwei parallel zur Mittelachse M verlaufende, zur Unterseite 18 hin geöffnete Nuten 24 vorgesehen. Die Nuten 24 dienen dem Eingriff von Stangen eines in den Fig. 3a und 3b beschriebenen Ständers.

In die Außenseite des Gehäuses 14 ist eine parallel zur Mittelachse M verlaufende, zur Oberseite 16 hin geöffnete Einlageaufnahme 30 eingebracht. Eine Beschriftungseinlage kann zur sicheren Kennzeichnung des Trägers 10 in diese Einlageaufnahme 30 eingesteckt werden.

An der Oberseite 16 ist ein ausziehbarer Tragegriff 26 vorgesehen. Der Tragegriff 26 ist hier im versenkten Zustand gezeigt, in dem er in einer zentral angeordneten Versenkungsöffnung 28 eingeschoben ist, um Platz zu sparen. Zum bequemeren Transport des Trägers 10 aus der Versenkungsöffnung 28 kann der Tragegriff 26 herausgezogen werden.

Ferner sind auf der Oberseite 16 des Gehäuses 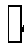 4 acht Vertiefungen 32 vorgesehen, die zur eindeutigen Kennzeichnung jeder Aufnahme 20 jeweils paarweise zwischen zwei Ladeöffnungen 20a angeordnet sind. Dazu zeigen jeweils die zwei Vertiefungen 32, die unmittelbar an derselben Ladeöffnung 20a anliegen, denselben Buchstaben A, B, C oder D. An Stelle der Buchstaben können auch Zahlen, Symbole oder Farben verwendet werden.

Benachbart zu jedem der vier Paare von Vertiefungen 32 ist an der Oberseite 16 radial weiter außen jeweils eine Ausnehmung 34 vorgesehen. Zur Ausnehmung 34 ist an der Unterseite 18 des Gehäuses 14 ein Vorsprung 36 angeordnet. Wenn mehrere Träger 10 aufeinander gestapelt werden, greifen die Vorsprünge 36 in die Ausnehmungen 34 ein, und es entsteht eine formschlüssige Verbindung zwischen den Vorsprüngen 36 und den Ausnehmungen 34. Dadurch sind mehrere Träger 10 stabil übereinander stapelbar.

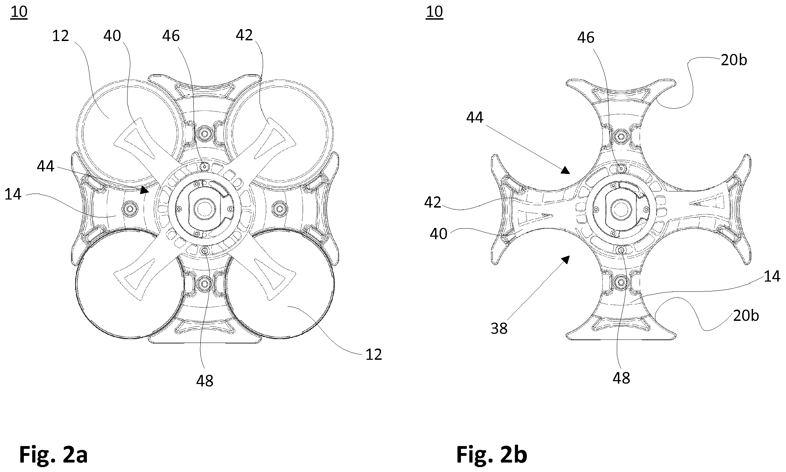

An der Unterseite 18 des Gehäuses 14 sind Schieber 40, 42 zum Öffnen und Schließen der Entladeöffnungen 20b angeordnet. Die Schieber 40, 42 werden in den Figuren 2a und 2b genauer beschrieben.

Fig. 2a zeigt eine Ansicht des Trägers 10 von unten mit in die Aufnahmen 20 geladenen Petrischalen 12 und einem in einer Schließposition befindlichen Verschließmechanismus 38, wobei die Petrischalen 12 von den Schiebern 40, 42 überdeckt werden.

Die Schieber 40, 42 sind paarweise und jeweils versetzt zueinander konzentrisch zur Mittelachse M des Gehäuses 14 angeordnet. Die Schieberpaare 40, 42 sind über ein Getriebe 44 gekoppelt, insbesondere in der Art eines Planetengetriebes, und mit einem Antrieb verbindbar. Das Getriebe 44 umfasst Zahnräder 46, 48, die in Eingriff mit den Schieberpaaren 40, 42 stehen. Die Zahnräder 46, 48 sind auf einem Grundkörper montiert und werden durch den Antrieb in Drehung versetzt. Durch die Drehung des Antriebs werden die Schieberpaare 40, 42 von einer Öffnungsposition in eine Schließposition überführt, die durch Anschläge begrenzt sind. Als Betätigungsmittel kann eine im Grundkörper zentrierte Vorrichtung vorgesehen sein, welche aus der Zentrierung und einem Ausleger besteht und vorzugsweise mit einem Antrieb verbunden ist, der sich insbesondere an einer Be-und Entladestation befindet.

Fig. 2b zeigt eine Ansicht des Trägers 10 von unten im unbeladenen Zustand, in dem sich die Schieberpaare 40, 42 in ihrer Öffnungsposition befinden und die Entladeöffnungen 20b freigegeben sind.

In Fig. 3a ist der Träger 10 im mit Petrischalen 12 beladenen Zustand, analog zu Fig. 2a, vor dem Einsetzen in einen Ständer 50 dargestellt.

Im Ständer 50 sind für die stabile Lagerung eines Stapels von Petrischalen 12 jeweils drei Stangen 52a, 52b, 52c vorgesehen, die bezogen auf den Umfang der Petrischalen 12 gleichmäßig voneinander beabstandet sind. Jeweils zwei Stangen 52a, 52b greifen beim in das Magazin eingesetzten Träger 10 in die in Fig. 1 beschriebenen Nuten 24 ein, die in der Wandung 22 der jeweiligen Aufnahme 20 eingebracht sind. Die jeweils dritte Stange 52c ist an der Stelle positioniert, an der die zugeordnete Aufnahme 20 auf der von der Mittelachse M weg weisenden Seite geöffnet ist.

In Fig. 3b ist der Träger 10 im entladenen Zustand, analog zu Fig. 2b, nach dem Einsetzen in den Ständer 50 dargestellt. Die Petrischalen 12 lagern nun zwischen den Stangen 52a, b.

Auf Grund der von der Ladeöffnung 20a entfernten Anordnung der Entladeöffnung 20b an der Unterseite 18 des Trägers 10 können mehrere vollständige Stapel von Petrischalen 12, beispielsweise nach dem Transport von einem Lagerort zu einer Analysevorrichtung, gleichzeitig in einen Ständer 50 eingesetzt und dort belassen werden, nachdem ein das Getriebe 44 und die Zahnräder 46, 48 umfassender Verschließmechanismus betätigt wurde und die Schieber 40, 42 in eine Öffnungsposition überführt wurden. Die Stabilität der Stapelanordnung wird zu keiner Zeit gefährdet, und die Petrischalen 12 verbleiben nach dem Entfernen des Trägers 10 im Ständer 50.

### Bezugszeichenliste

- 10: Träger
- 12: Petrischalen
- 14: Gehäuse
- 16: Oberseite
- 18: Unterseite
- 20: Aufnahmen
- 20a: Ladeöffnung
- 20b: Entladeöffnung
- 22: Wandung
- 24: Nut
- 26: Tragegriff
- 28: Versenkungsöffnung
- 30: Einlagenaufnahme
- 32: Vertiefung
- 34: Ausnehmung
- 36: Vorsprung
- 38: Verschließmechanismus
- 40, 42: Schieber
- 44: Getriebe
- 44a: Verzahnung
- 46, 48: Zahnräder
- 50: Ständer
- 52a, b, c: Stangen
- M: Mittelachse
- L: Ladeachse

## Patentansprüche

1. Träger (10) zum Aufnehmen und Lagern von Laborgefäßen für Proben, Mikroorganismen, Zellkulturen, oder ähnlichem, mit einem Gehäuse (14), zumindest einer entlang einer Ladeachse (L) im Gehäuse (14) sich erstreckenden Aufnahme (20) für die Laborgefäße, in welche die Laborgefäße einbringbar und darin übereinander stapelbar sind, wobei die Aufnahme (20) im Gehäuse (14) zum Beladen eine Ladeöffnung (20a) aufweist, wobei die Aufnahme (20) auf einer der Ladeöffnung (20a) entfernt angeordneten Unterseite (18) eine mittels eines im Gehäuse (14) angeordneten Verschließmechanismus (38) verschließbare Entladeöffnung (20b) aufweist, wobei der Verschließmechanismus (38) mit einem Schieber (40, 42) versehen ist, wobei der Schieber eine Sperrarmatur ist, welche in einer Ebene bewegt wird, die im Wesentlichen senkrecht zum zu sperrenden Materialdurchfluss verläuft, und wobei der Schieber (40, 42) zum Schließen in die Entladeöffnung (20b) in eine Schließposition und zum Öffnen aus der Entladeöffnung (20b) in eine Öffnungsposition bewegbar ist, **dadurch gekennzeichnet, dass** der Verschließmechanismus (38) den Schieber (40, 42) aus der Schließposition in die Öffnungsposition und vice versa rotatorisch bewegt.

2. Träger nach Anspruch 1, **dadurch gekennzeichnet, dass** ein Getriebe vorgesehen ist, das mit dem Schieber (40, 42) zusammenwirkt.

3. Träger nach Anspruch 1 oder 2, **dadurch gekennzeichnet, dass** mehrere Aufnahmen (20) mit zugeordneten Lade- und Entladeöffnungen (20a, 20b) vorgesehen sind, und jeder Entladeöffnung (20b) ein Schieber (40, 42) zugeordnet ist, insbesondere dass die Schieber (40, 42) so miteinander gekoppelt sind, dass über einen Antrieb alle Schieber (40, 42) gleichzeitig bewegbar sind.

4. Träger nach einem der vorangehenden Ansprüche, **dadurch gekennzeichnet, dass** der Verschließmechanismus (44, 46, 48) von der Unterseite (18) des Trägers (10) betätigbar ist, auf der die Entladeöffnung (20b) angeordnet ist.

5. Träger nach einem der vorangehenden Ansprüche, **dadurch gekennzeichnet, dass** der Verschließmechanismus (44, 46, 48) an der Unterseite (18) eine Antriebsnabe oder einen Mitnehmer aufweist, über die der Antriebsmechanismus motorisch oder manuell, insbesondere mittels einer an die Antriebsnabe oder den Mitnehmer angreifenden Handhabe, betätigbar ist.

6. Träger nach einem der vorangehenden Ansprüche, **dadurch gekennzeichnet, dass** die Aufnahme (20) eine kreisförmige oder zumindest teilkreisförmige Grundfläche aufweist,

7. Träger nach einem der vorangehenden Ansprüche, **dadurch gekennzeichnet, dass** die Ladeöffnung (20a) und Entladeöffnung (20b) konzentrisch zueinander angeordnet sind, so dass die Ladeachse (L) sich senkrecht zu der Ladeöffnung (20a) und der Entladeöffnung (20b) erstreckt und insbesondere parallel zu einer Längsachse des Trägers verläuft.

8. Träger nach einem der vorangehenden Ansprüche, **dadurch gekennzeichnet, dass** ein an einer Oberseite (16) des Gehäuses (14) angeordneter Tragegriff (26) vorgesehen ist, der insbesondere im Gehäuse (14) versenkbar gelagert ist, insbesondere der Tragegriff (26) vollständig im Gehäuse (14) versenkbar ist, so dass dieser nicht über eine Umhüllende des Gehäuses (14) hinaussteht.

9. Träger nach einem der vorangehenden Ansprüche, **gekennzeichnet durch** einen hitzebeständigen Werkstoff, der eine Autoklavierung des Trägers (10) ermöglicht.

10. Träger nach einem der vorangehenden Ansprüche, **dadurch gekennzeichnet, dass** entlang der Längsachse der Aufnahme (20) für die Laborgefäße wenigstens zur Unterseite (18) hin offene Ablaufnuten vorgesehen sind.

11. Träger nach einem der vorangehenden Ansprüche, **dadurch gekennzeichnet, dass** zumindest im Bereich der Entladeöffnung (20b) und/oder Ladeöffnung (20a) Eingriffsausnehmungen (24) vorgesehen sind, in die Ständer (50) einfahrbar sind, welche mit Abziehen des Trägers die Laborgefäße aufnehmen können.

12. Träger nach einem der vorangehenden Ansprüche, **dadurch gekennzeichnet, dass** die Eingriffsausnehmungen (24) als parallel zur Längsachse ausgerichtete Durchgangsbohrungen oder als zu der Aufnahme (20) für die Laborgefäße hin offene, seitliche Nuten ausgebildet sind.

13. Träger nach einem der vorangehenden Ansprüche, **dadurch gekennzeichnet, dass** zueinander korrespondierende Vorsprünge (36) und Ausnehmungen (34) an der Oberseite (16) und Unterseite (18) vorgesehen sind, so dass die Vorsprünge (36) und Ausnehmungen (34) bei mehreren gestapelten Trägern ineinandergreifen und dadurch im Wesentlichen normal zur Längsachse formschlüssig fixiert sind, insbesondere dass die Vorsprünge (36) und zugeordneten Ausnehmungen (34) so ausgebildet sind, dass eine Stapelung mehrerer Gehäuse (14) von Trägern in konzentrischer Weise zueinander ermöglicht wird.

14. Träger nach einem der vorangehenden Ansprüche, **gekennzeichnet durch** eine Ausbildung des Gehäuses (14) als in einem Kunststoff-Spritzgussverfahren hergestelltes Teil, insbesondere als Vollgussteil.

15. Träger nach einem der vorangehenden Ansprüche, **dadurch gekennzeichnet, dass** seitlich im Gehäuse (14) eine Einlagenaufnahme (30) für eine Beschriftungseinlage vorgesehen ist.

## Claims

1. Rack (10) for receiving and storing laboratory vessels containing samples, microorganisms, cell cultures or the like, which rack (10) comprises a housing (14), at least one laboratory vessel holder (20) that extends along a loading axis (L) in the housing (14) and which is adapted for inserting and vertically stacking the laboratory vessels therein, with a loading opening (20a) being provided in the housing (14) for loading vessels into the holder (20), and an unloading opening (20b) being provided on an underside (18) distally located from said loading opening (20a) for unloading vessels from the holder (20), which unloading opening (20b) can be closed by means of a closing mechanism (38) arranged in the housing (14), which closing mechanism (38) is provided with a slide (40, 42), said slide being a shut-off fitting that is adapted to be moved in a plane substantially perpendicular to the material flow to be shut off, and which slide (40, 42) can be moved to a closing position located within the discharge opening (20b) for blocking the opening, and can be moved to an opening position located away from the discharge opening (20b) for releasing the opening, **characterized in that** said closing mechanism (38) is used to rotate the slide (40, 42) from the closing position into the opening position and vice versa.

2. Rack according to claim 1, **characterized in that** a gear mechanism is provided which interacts with the slide (40, 42).

3. Rack according to claim 1 or 2, **characterized in that** a plurality of holders (20) having respective loading and unloading openings (20a, 20b) are provided, and that a respective slide (40, 42) is provided for each unloading opening (20b), in particular **in that** the slides (40, 42) are coupled to one another in such a way that all the slides (40, 42) can be moved simultaneously via a drive.

4. Rack according to any one of the preceding claims, **characterized in that** the closing mechanism (44, 46, 48) can be operated from the underside (18) of the rack (10) where the unloading opening (20b) is located.

5. Rack according to any one of the preceding claims, **characterized in that** on its underside (18), the closing mechanism (44, 46, 48) has a drive hub or a driver via which the drive mechanism can be operated by motor or manually, in particular by means of a handle engaging the drive hub or the driver.

6. Rack according to any one of the preceding claims, **characterized in that** the base area of the holder (20) is circular or at least partially circular.

7. Rack according to any one of the preceding claims, **characterized in that** the loading opening (20a) and the unloading opening (20b) are arranged concentrically to one another such that the loading axis (L) extends perpendicularly with respect to the loading opening (20a) and the unloading opening (20b) and in particular is parallel to a longitudinal axis of the rack.

8. Rack according to any one of the preceding claims, **characterized in that** a carrying handle (26) is provided which is arranged on an upper side (16) of the housing (14), which handle (26) is in particular mounted so as to be retractable into the housing (14), that in particular the carrying handle (26) is completely retractable into the housing (14) so that it does not protrude beyond an envelope of the housing (14).

9. Rack according to any one of the preceding claims, **characterized by** a heat-resistant material that will withstand autoclaving of the rack (10).

10. Rack according to any one of the preceding claims, **characterized in that** along the longitudinal axis of the holder (20) for the laboratory vessels, drain grooves are provided that are open at least towards the underside (18).

11. Rack according to any one of the preceding claims, **characterized in that** engagement openings (24) are provided at least in the area of the unloading opening (20b) and/or the loading opening (20a), with posts (50) being introduced into said engagement openings (24) so as to accommodate the laboratory vessels once the rack is removed.

12. Rack according to any one of the preceding claims, **characterized in that** the engagement openings (24) are formed as through bores aligned parallel to the longitudinal axis, or as lateral grooves that are open towards the holder (20) for the laboratory vessels.

13. Rack according to any one of the preceding claims, **characterized in that** projections (36) and corresponding recesses (34) are provided on the upper side (16) and on the underside (18) in such a manner that the projections (36) and recesses (34) will engage one another when several racks are vertically stacked, and are thereby positively secured substantially perpendicular to the longitudinal axis, in particular that the projections (36) and associated recesses (34) are designed in a way that will allow a plurality of rack housings (14) to be stacked concentrically relative to one another.

14. Rack according to any one of the preceding claims, **characterized by** a design of the housing (14) as a part produced in a plastic injection molding process, in particular as a solid injection-molded part.

15. Rack according to any one of the preceding claims, **characterized in that** an insert receiver (30) for a labelling insert is provided on the side of the housing (14).

## Revendications

1. Support (10) pour la réception et le stockage de récipients de laboratoire pour des échantillons, des micro-organismes, des cultures de cellules, ou similaires, avec un boîtier (14), au moins un logement (20) s'étendant le long d'un axe de chargement (L) dans le boîtier (14) pour les récipients de laboratoire, dans lequel les récipients de laboratoire peuvent être introduits et peuvent être empilés les uns sur les autres, dans lequel le logement (20) présente dans le boîtier (14), pour le chargement, une ouverture de chargement (20a), dans lequel le logement (20) présente sur une face inférieure (18) disposée à distance de l'ouverture de chargement (20a) une ouverture de déchargement (20b) pouvant être fermée au moyen d'un mécanisme de fermeture (38) disposé dans le boîtier (14), dans lequel le mécanisme de fermeture (38) est pourvu d'un coulisseau (40, 42), dans lequel le coulisseau est une armature de blocage, laquelle est déplacée dans un plan qui s'étend sensiblement perpendiculairement au débit de matériau à bloquer, et dans lequel le coulisseau (40, 42), aux fins de fermeture, peut être déplacé dans l'ouverture de déchargement (20b) dans une position de fermeture, et, aux fins d'ouverture, peut être déplacé à l'extérieur de l'ouverture de déchargement (20b) dans une position d'ouverture, **caractérisé en ce que** le mécanisme de fermeture (38) déplace en rotation le coulisseau (40, 42) de la position de fermeture dans la position d'ouverture et vice versa.

2. Support selon la revendication 1, **caractérisé en ce qu'**une transmission est prévue, qui coopère avec le coulisseau (40, 42).

3. Support selon la revendication 1 ou 2, **caractérisé en ce que** plusieurs logements (20) sont pourvus d'ouvertures de chargement et de déchargement (20a, 20b) associées, et un coulisseau (40, 42) est associé à chaque ouverture de déchargement (20b), en particulier que les coulisseaux (40, 42) sont accouplés l'un à l'autre, de sorte que tous les coulisseaux (40, 42) peuvent être déplacés simultanément par l'intermédiaire d'un entraînement.

4. Support selon l'une quelconque des revendications précédentes, **caractérisé en ce que** le mécanisme de fermeture (44, 46, 48) peut être actionné par la face inférieure (18) du support (10), sur laquelle l'ouverture de déchargement (20b) est disposée.

5. Support selon l'une quelconque des revendications précédentes, **caractérisé en ce que** le mécanisme de fermeture (44, 46, 48) présente sur la face inférieure (18) un moyeu d'entraînement ou un entraîneur, par l'intermédiaire duquel le mécanisme d'entraînement peut être actionné de manière motorisée ou manuelle, en particulier au moyen d'une manette agissant sur le moyeu d'entraînement ou l'entraîneur.

6. Support selon l'une quelconque des revendications précédentes, **caractérisé en ce que** le logement (20) présente une surface de base circulaire ou au moins en partie circulaire.

7. Support selon l'une quelconque des revendications précédentes, **caractérisé en ce que** l'ouverture de chargement (20a) et l'ouverture de déchargement (20b) sont disposées de manière concentrique l'une par rapport à l'autre, de sorte que l'axe de chargement (L) s'étend perpendiculairement à l'ouverture de chargement (20a) et à l'ouverture de déchargement (20b) et en particulier parallèlement à un axe longitudinal du support.

8. Support selon l'une quelconque des revendications précédentes, **caractérisé en ce qu'**une poignée de transport (26) disposée sur une face supérieure (16) du boîtier (14) est prévue, qui est montée de manière à pouvoir être enfoncée en particulier dans le boîtier (14), en particulier la poignée de transport (26) peut être enfoncée entièrement dans le boîtier (14), de sorte que celle-ci ne fait pas saillie d'une enveloppe du boîtier (14).

9. Support selon l'une quelconque des revendications précédentes, **caractérisé par** un matériau résistant à la chaleur, qui permet un autoclavage du support (10).

10. Support selon l'une quelconque des revendications précédentes, **caractérisé en ce que** des rainures d'évacuation ouvertes sont prévues le long de l'axe longitudinal du logement (20) pour les récipients de laboratoire au moins en direction de la face inférieure (18).

11. Support selon l'une quelconque des revendications précédentes, **caractérisé en ce que** des évidements d'insertion (24), dans lesquels des montants (50), lesquels peuvent loger les récipients de laboratoire avec retrait du support, peuvent être insérés au moins dans la zone de l'ouverture de déchargement (20b) et/ou ouverture de chargement (20a).

12. Support selon l'une quelconque des revendications précédentes, **caractérisé en ce que** les évidements d'insertion (24) sont réalisés sous la forme de trous de passage orientés parallèlement à l'axe longitudinal ou de rainures latérales ouvertes en direction du logement (20) pour les récipients de laboratoire.

13. Support selon l'une quelconque des revendications précédentes, **caractérisé en ce que** des parties saillantes (36) et des évidements (34) correspondants les uns aux autres sont prévus sur la face supérieure (16) et face inférieure (18), de sorte que les parties saillantes (36) et évidements (34) s'insèrent les uns dans les autres lorsque plusieurs supports sont empilés et sont ainsi fixés par coopération de formes sensiblement perpendiculairement à l'axe longitudinal, en particulier que les parties saillantes (36) et évidements (34) associés sont réalisés de sorte qu'un empilement de plusieurs boîtiers (14) de supports de manière concentrique les uns par rapport aux autres est permis.

14. Support selon l'une quelconque des revendications précédentes, **caractérisé par** une réalisation du boîtier (14) sous la forme d'une pièce fabriquée au cours d'un procédé de moulage par injection de matière plastique, en particulier sous la forme d'une pièce entièrement coulée.

15. Support selon l'une quelconque des revendications précédentes, **caractérisé en ce qu'un** logement d'insert (30) pour un insert d'inscription est prévu latéralement dans le boîtier (14).
